# EUROPÄISCHE PATENTSCHRIFT

(11) **EP 2 484 503 B1**
(45) Veröffentlichungstag und Bekanntmachung des Hinweises auf die Patenterteilung: **16.04.2014**
(21) Anmeldenummer: 12153852.4
(22) Anmeldetag: 03.02.2012
(51) Int. Cl.: B27L 7/00, B27L 7/06, B25D 3/00, B26B 3/00, B26B 23/00

(54) **Handbetätigtes Schlagwerkzeug zum Spalten von Holzbriketts**
Manually actuated striking tool for splitting wood briquettes
Outil de percussion manuel pour fendre de briquettes de bois

(30) Priorität: 04.02.2011 AT 1542011
(43) Veröffentlichungstag der Anmeldung: 08.08.2012
(73) Patentinhaber: Gilow, Alfred, 5020 Salzburg (AT)
(72) Erfinder: Gilow, Alfred, 5020 Salzburg (AT)
(74) Vertreter: Ganahl, Bernhard

(56) Entgegenhaltungen:
- WO-A1-2009/108977
- CH-A- 245 451
- DE-A1- 1 923 349
- DE-U1- 20 109 639
- DE-U1-202004 014 162
- DE-U1-202009 000 235
- FR-A- 888 511
- FR-A1- 2 256 005
- FR-A1- 2 431 351
- US-A- 111 024
- US-A- 881 538
- US-A- 1 521 265
- US-A- 3 187 354
- US-A- 3 377 052
- US-A1- 2007 256 305
- US-A1- 2009 056 407
- US-A1- 2011 244 772

## Beschreibung

### Beschreibung

Die Erfindung betrifft ein handbetätigtes Schlagwerkzeug zum Spalten handelsüblicher Holzbriketts unterschiedlicher Ausführung senkrecht zu deren Längsachse.

Unter manuellen Schlagwerkzeugen versteht man im allgemeinen Hämmer, Äxte und Beile. Eine Untergruppe stellen die Spaltwerkzeuge dar, und hier wiederum diejenigen, welche zum Spalten von Holz geeignet sind. Allen diesen Spaltwerkzeugen ist eine für ihren speziellen Zweck ausgeformte Klinge, ein entsprechendes Gewicht zur Freisetzung der beim Hub gespeicherten kinetischen Energie bei der Schlagausführung und ein der jeweiligen Funktion angepasster Stiel zur Übertragung der Muskelkraft auf das Werkzeug gemein.

Das Spalten von Holz war bislang Werkzeugen wie Äxten, Beilen und Keilen in Kombination mit entsprechenden Schlagwerkzeugen vorbehalten. Seit der Entwicklung neuer, auf dem Werkstoff "Holz" basierender Brennstoffe, hier insbesondere sog. "Holzbriketts", wurde - bis auf eine Ausnahme (DE 202004010275U1) - jedoch kein Werkzeug zur Spaltung eben dieser Holzbriketts entwickelt, obwohl, der Bedarf gegeben wäre.

In der DE 20 2004 014 162 U1 wird ein handbetätigtes Schlagwerkzeug beschrieben das geeignet ist zum Spalten von Holzbriketts umfassend einen balligen Griff und zumindest eine Handschutzscheibe, die an einem Ende des Griffs angeordnet ist.

In der DE 202004010275U1 wird eine Zange beschrieben, welche zum Spalten von Holzbriketts gedacht ist. Als nachteilig erweist sich, dass diese Zange aufgrund der hierbei zur Anwendung gelangenden Hebelgesetze nur mit hohem Kraftaufwand zum Einsatz gelangt, Verschleißteile aufweist, an unterschiedliche Durchmesser der Holzbriketts angepasst werden muss und somit insgesamt die Dauer des Spaltprozesses verzögert wird. Eine Verwendung bei Holzbriketts mit anderem als rundem Querschnitt ist ausgeschlossen. Die Verpackungsfolie von Holzbrikett-Gebinden lässt sich mit der Zange nicht entfernen.

In der JP 3128083 A wird ein Küchenmesser beschrieben, welches eine massive Einheit zwischen Klinge und Griff bildet.

In der FR 2805980 A1 wird ein Werkzeug zum Öffnen von Austernschalen beschrieben.

Der Erfindung liegt die Aufgabe zugrunde, ein handbetätigtes Schlagwerkzeug zum Spalten von Holzbriketts zu schaffen, mit welchem einfach, sicher und zuverlässig Holzbriketts gespaltet werden können.

Die Erfindung wird durch ein handbetätigtes Schlagwerkzeug zum Spalten von Holzbriketts gemäß Anspruch 1 gelöst. Vorteilhafte Ausgestaltungen sind in den Unteransprüchen angegeben.

Das erfindungsgemäße handbetätigte Schlagwerkzeug zum Spalten von Holzbriketts senkrecht zu deren Längsachse zeichnet sich dadurch aus, dass es mit einem aus Stahl oder Gusseisen gefertigten, balligen Griff ausgebildet ist, an dessen Ende zumindest eine Handschutzscheibe angebracht ist, die ebenfalls aus Stahl oder Gusseisen gefertigt ist, und einer an der Unterseite der Handschutzscheibe angebrachten, kurzen, ballig-konischen, in einer Spitze auslaufenden Klinge, wobei das Schlagwerkzeug ein Gewicht von zumindest 300 g aufweist.

Die Erfindung eines "Holzbrikettspalters" kann unter dem Oberbegriff "Schlagwerkzeug" insofern eingereiht werden, als hier - wie bei anderen Schlagwerkzeugen - kinetische Energie in Verbindung mit Muskelkraft zum Einsatz kommt. Als weitere Differenzierung eignet sich hier die Untergruppe der "Spaltwerkzeuge" für das Spalten von Holz insofern, als durch die Erfindung das Spalten eines aus Holz bestehenden Werkstoffes in Form eines Holzbriketts ermöglicht wird.

Als innovativ zu erkennen ist bei der vorgestellten Erfindung - im Gegensatz zu anderen manuellen Spaltwerkzeugen - die Zusammenführung von Klinge und Griff unter Weglassung eines Stieles zu einer kompakten Schlageinheit, welche ihre kinetische Energie aus der Besonderheit ihres auf Gewichtmaximierung ausgelegten Griffes und dessen Handschutzscheiben bezieht. Ein signifikantes Unterscheidungsmerkmal zu anderen Schlag - bzw. Spaltwerkzeugen besteht folglich darin, dass hier das zum Schlag erforderliche Gewicht vor allem im Griff und nicht in der Klinge anzutreffen ist.

Ein weiteres Unterscheidungsmerkmal zu anderen Spaltwerkzeugen für Holzwerkstoffe besteht in der besonderen Ausformung der Klingengeometrie, welche insbesondere dadurch gekennzeichnet ist, dass sie - direkt unterhalb des Griffes angebracht - eine ballig - konische, zu einer Spitze auslaufende Form aufweist und wegen ihrer kurzen Dimension und ihrer balligen Form funktionsbestimmende Scherkräfte beim Spaltvorgang entwickelt und darüber hinaus eine besondere - durch ihre Kürze bedingte - Zielsicherheit beim Spaltvorgang aufweist. Dies ermöglicht auch Personen, die nicht gewohnt sind. Mit einer Axt umzugehen, das zuverlässige und sichere Spalten eines Holzbriketts.

Zudem ist das Spaltwerkzeug durch das Weglassen eines Stiels sehr kompakt ausgebildet, was für die Anwendung im Haushalt von Vorteil ist.

Das Verständnis der vorgestellten Erfindung in Form eines Holzbrikettspalters erschließt sich jedoch erst bei genauerer Betrachtung des zu bearbeitenden Werkstoffes eines Holzbriketts.

### Beschreibung des zu bearbeitenden Werkstoffes

Holzbriketts werden im Handel vornehmlich als Brennstoff unterschiedlicher Sorten für Kaminöfen u.ä. angeboten und weisen in der Regel eine zylindrische Form mit einer Länge von 270mm und einen Durchmesser von 95 mm auf. Wegen ihrer bequemen Lagerhaltung, ihrer einfachen Anwendung und ihrer ökonomischen wie ökologischen Vorteile erfreuen sie sich zunehmender Beliebtheit.

Sie bestehen aus axial verpressten Hobel- und Sägespänen, welche aufgrund der bei dem Pressvorgang entstehenden Wärme mit dem im Holz frei werdenden Lignin verbacken sind. Der Pressvorgang erzeugt eine senkrecht zur Achse des Holzbriketts ausgerichtete Schichtstruktur, auf welche im weiteren Verlauf der Darstellung Bezug genommen wird.

Üblichweise gelangen die Holzbriketts in Verpackungseinheiten von jeweils fünf in PE-Folie verschweißten Einheiten in den Handel. Auch auf diese Verpackungsart wird im weiteren Verlauf Bezug genommen.

Trotz der beschriebenen Vorteile von Holzbriketts weisen diese auch gewisse Nachteile auf:
- Handelsübliche Brennkammern von Kaminöfen sind ihrer Dimensionierung nach nicht für die Länge der beschriebenen Holzbriketts ausgelegt, insbesondere deshalb, weil sich Holzbriketts bei Erhitzung axial um ca. 100% ausdehnen uns somit die Dimension der meisten Brennkammern überschreiten.
- Die Regulierung der Heizleistung von Kaminöfen erfolgt einerseits durch eine dosierte Zufuhr von Sauerststoff durch einen Schieber, andererseits durch die Menge des zugeführten Brennstoffes - hier in Form von Holzbriketts.

Zur Wärmeregulierung ist es demnach unumgänglich, die Holzbriketts zu portionieren, indem man sie spaltet.
- Das Anfeuern von Holzbriketts im Stück erweist sich als problematisch, da ein Anschürfeuer zu wenig entzündbare Oberfläche vorfindet. Auch hier erweist es sich von Vorteil, eine entsprechende Menge an Holzbrikett in ca. 2-3 cm dicke Scheiben zu spalten, um sie pyramidenartig in der Brennkammer auf dem Rost über einen Anzündstoff (in der Regel Papier oder in Wachs getränkte Holzwolle) aufschichten zu können. Der Anschürvorgang wird durch dieses Verfahren wesentlich vereinfacht.
- Holzbriketts sind üblicherweise in Verpackungseinheiten zu je fünf Stück in PE-Folie eingeschweißt. Es bedarf demnach eines zusätzlichen Werkzeuges in Form einer scharfen Klinge, um die Folie zu entfernen. Die vorgestellte Erfindung weist funktionsbedingt eine scharfe Klinge auf, welche ebenso zum Aufschneiden der PE-Folie geeignet ist.

### Stand der Technik

### Ausführungsbeispiel

Die Erfindung wird nun unter Bezugnahme auf ein Ausführungsbeispiel, welches in der Zeichnung schematisch dargestellt ist, weiter erläutert.

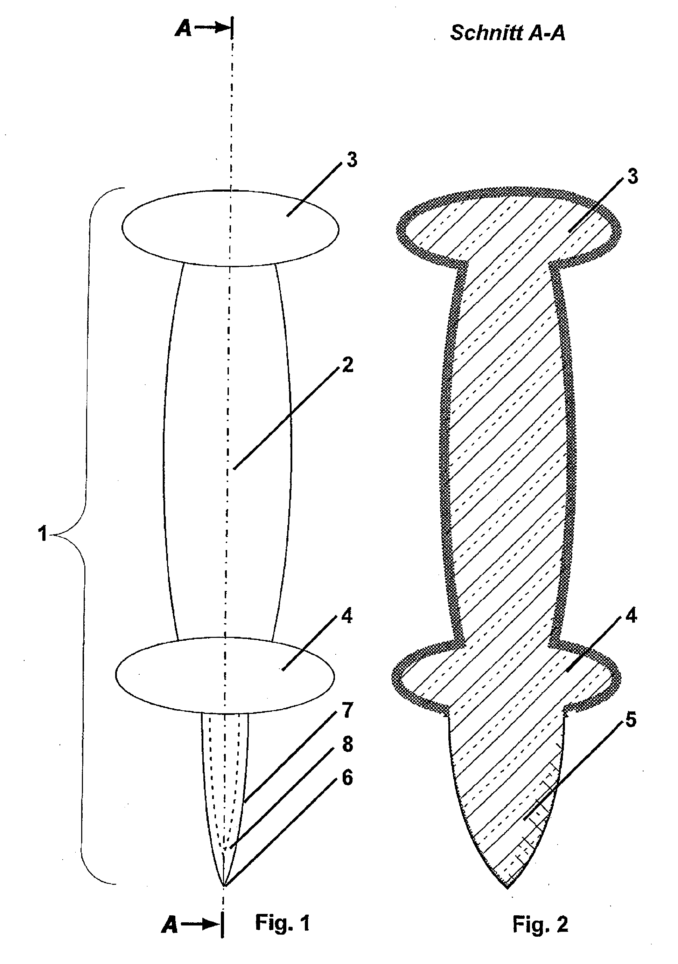

Fig. 1 stellt in Seitenansicht den Holzbrikettspalter dar,
Fig. 2 zeigt den Holzbrikettspalter im Längsschnitt der Vorderansicht,

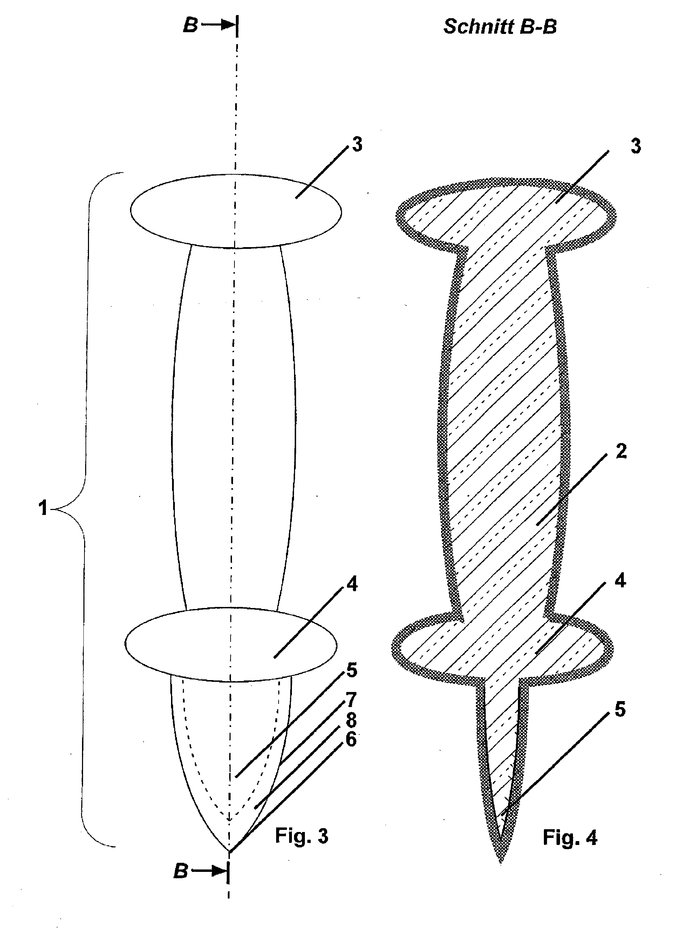

Fig. 3 stellt in Vorderansicht den Holzbrikettspalter dar,
Fig. 4 zeigt den Holzbrikettspalter im Längsschnitt der Seitenansicht,
Fig. 5 und 6 zeigen eine Spalterschütte jeweils in einer Schnittdarstellung.

Wie eingangs schon erwähnt, ist beim Befeuern von Kaminöfen mit Holzbriketts eine Portionierung durch Spaltung unerlässlich, sei es, um die Menge des einzubringenden Brennmaterials zu dosieren, oder um den Anschürvorgang mittels radial abgespalteter - dem Pressvorgang entsprechender - Scheiben zu optimieren.

Mit der Erfindung wird erreicht, dass sich ein Holzbrikett mit geringem Kraft- und Zeitaufwand in beliebig große Stücke oder Scheiben senkrecht zur Längsachse des Holzbriketts spalten lässt.

Ein handelsübliches Messer ist für diese Aufgabe ungeeignet, da ihm - mangels Masse - die für den Spaltvorgang notwendige kinetische Energie fehlt und außerdem die Schneidengeometrie für derlei Spaltvorgänge ungeeignet ist.

Ausschlaggebend für die Funktion des handbetätigten Schlagwerkzeugs (Holzbrikettspalters) 1 sind im Wesentlichen drei Merkmale, welche eine Funktionseinheit ergeben:
1. Ein aus schwerem Material (Stahl, bzw. Gusseisen) gefertigte, ergonomisch ballig ausgeführter Griff 2 erhöht durch sein Gewicht die Schlagkraft der führenden Hand beim Schlagen auf das zu spaltende Holzbrikett senkrecht zu dessen Längsachse maßgeblich.
2. Zumindest an einem Ende des Griffs 2 ist eine Handschutzscheibe 3, 4 angebracht.
3. An den Griff 2, vorzugsweise angrenzend an der zumindest einen Handschutzscheibe 4, ist unmittelbar eine Spaltklinge 5 angebracht.

Die Spaltklinge läuft in einer Spitze 6 zusammen und nimmt zu einer mit der Handschutzscheibe 4 verbundenen Basis hin ballig-konisch (konvex) zu. Die seitlichen Klingenkanten 7, ebenfalls von der Spitze zur Basis hin ballig-konisch (konvex) verlaufend, sind hierbei zu zwei Schneidekanten 8 beidseitig geschliffen, wodurch sich eine scharfe, pfeilartige Spitze 6 ergibt. Die Spaltklinge 5 ist vorzugsweise an ihrer ballig-konischen Basis um das ca. 0,2-fache seiner Länge größer und zur Optimierung des Spaltvorganges weist die Breite das ca. 0,7-fache seiner Länge auf, um mit den dadurch beim Schlag zum Einsatz kommenden Scherkräften eine optimale Spaltwirkung zu erzielen. Vorteilhaft ist hierbei insbesondere die kurze Dimensionierung der Klinge 5 von nicht mehr als 60 mm, insbesondere nicht mehr als 50 mm bzw. 45 mm. Andererseits sollte die Klinge nicht kürzer als 20 mm und insbesondere nicht kürzer als 30 mm bzw. 40 mm sein. Durch die kurze Länge der Klinge 5 sind einerseits die Scherkräfte optimiert und andererseits ist durch die kurze Distanz zwischen Schlaghand und Klinge die Zielgenauigkeit bei der Schlagausführung gewährleistet. Zudem besitzt eine derart kurze Klinge eine hohe Stabilität, die dem Schlagwerkzeug eine lange Lebensdauer verleit.

An der Basis besitzt die Klinge eine Bereite von 25 mm bis 45 mm und insbesondere von 30 mm bis 40 mm. Die Dicke an der Basis im Zentrum der Klinge beträgt 4 bis 10 mm und insbesondere 5 bis 8 mm.

Die Schneidekanten 8 dienen zusätzlich zum Aufschneiden von PE-Schutzfolien, in welche die Verpackungseinheiten handelsüblicher Holzbrikettgebinde eingeschweißt sind. Ein zusätzliches Werkzeug wird für diesen Vorgang somit nicht benötigt.

Das den Spaltvorgang unterstützende Eigengewicht des Holzbrikettspalters 1 von zumindest 300 g wird in Addition durch das Gewicht des Griffes 2, in massiver Bauweise gefertigt (Stahl, bzw. Gusseisen), durch das Gewicht zumindest einer ebenfalls in massiver Bauweise (Stahl, bzw. Gusseisen) gefertigten Handschutzscheibe 3, 4 und das einer Klinge 5 erzielt. Die Handschutzscheiben weisen hierbei einen Durchmesser von zumindest 50 mm bzw. zumindest 60 mm auf und der Durchmesser liegt vorzugsweise in einem Bereich von 50 mm - 80 mm bzw. 60 mm - 70 mm. Die Dicke der Handschutzscheibe beträgt etwa zumindest 15 mm und bevorzugt zumindest 20 mm und liegt insbesondere im Bereich von 20 mm - 25 mm. Eine Form der Handschutzscheiben 3, 4, die einem Ellipsenabschnitt entspricht ist ergonomisch vorteilhaft.

Ein wesentlicher Bestandteil der Erfindung besteht folglich in dem konstruktiven Merkmal der Kombination aus Griff 2 und der Handschutzscheibe 4.

Das dadurch erzielte Gewicht des Holzbrikettspalters ist ausschlaggebend für die Aufnahme der kinetischen Energie und somit für dessen Funktion.

Das für den Spaltvorgang optimale Gesamtgewicht des Spalters 1 sollte, gemäß empirischer Versuchsreihen anhand von div. Prototypen, zumindest 300 g betragen. Das Gesamtgewicht beträgt vorzugsweise zumindest 350 g bzw. 400 g bzw. 450 g bzw. 500 g und insbesondere zumindest 550 g.

Damit das Spaltwerkzeug einfach handhabbar ist, sollte sein Gewicht nicht mehr als 1000 g, insbesondere nicht mehr als 900 g bzw. 800 g bzw. 700 g betragen.

Das Schlagwerkzeug weist vorzugsweise zumindest im Bereich des Griffes 2 und vorzugsweise im Bereich der Handschutzscheiben 3 und 4 einen Kunststoffüberzug auf. Der Kunststoffüberzug ist beispielsweise ein Neoprenüberzug. Ein solcher Neoprenüberzug kann mittels eines Tauchverfahrens aufgebracht werden.

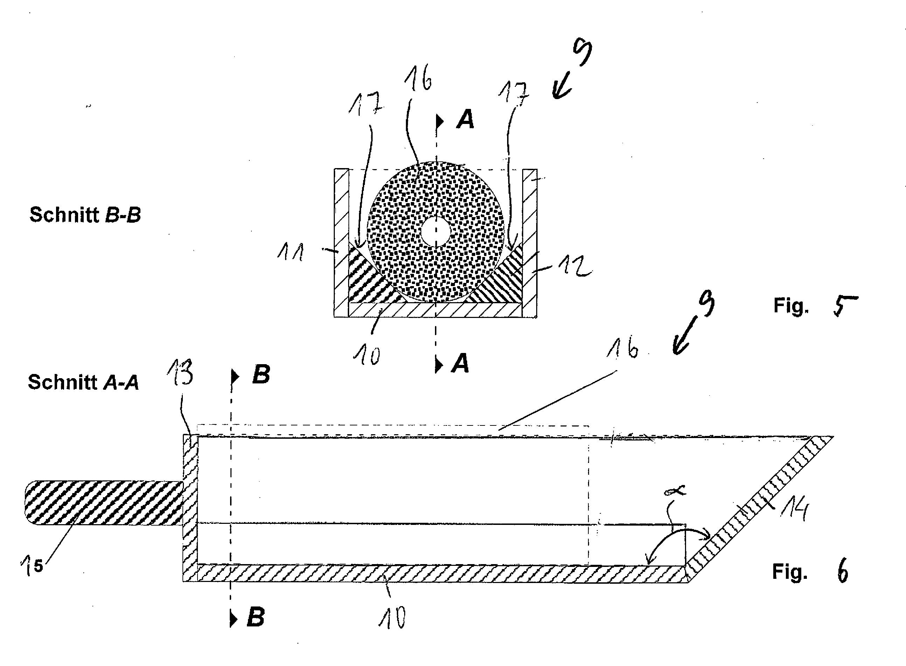

Figur 5 und 6 zeigen eine Spalterschütte 9. Die Spalterschütte 9 weist eine Bodenwandung 10, zwei Seitenwandungen 11, 12, eine Rückwandung 13 und eine Vorderwandung 14 auf. Diese Wandungen begrenzen einen langgestreckten, einseitig offenen Hohlraum. Die einzelnen Wandungen sind im rechten Winkel zueinander angeordnet bis auf die Vorderwandung 14, die gegenüber der Bodenwandung 10 schräg angeordnet ist und mit dieser einen Winkel α von etwa 120°bis 150°einschließt.

An der Rückwandung 13 ist ein nach außen hervorstehender Griff 15 befestigt. In die durch die Bodenwandung 10 und die jeweiligen Seitenwandungen 11, 12 begrenzten Eckbereiche ist jeweils eine im Querschnitt dreieckförmige Leiste eingesetzt, so dass diese Leiste bündig an der Bodenwandung 10 und an der jeweiligen Seitenwandung 11, 12 anliegt und mit ihrer freien Seite eine in der Spalterschütte 9 schräg verlaufende Begrenzungsfläche 17 derart bildet, dass eine im Querschnitt kreisförmiges Holzbrikett 16 in die Spalterschütte 9 eingelegt werden kann, ohne hierin seitlich weg zu rollen. Diese beiden schräg verlaufenden Begrenzungsflächen 17 erlauben es, dass im Querschnitt kreisförmige Holzbrikette mit unterschiedlichem Durchmesser eingelegt werden können.

Die Wandungen der Spalterschütte 9 sind aus einem stabilen Material, insbesondere einem Hartholz oder Metall, wie zum Beispiel Gusseisen, Stahl oder Stahlblech, ausgebildet, so dass die Spalterschütte 9 nicht beim Zerteilen eines Holzbriketts 16 mittels des Spaltwerkzeuges 1 beeinträchtigt wird. Vorzugsweise umfasst ein System zum Spalten von im Querschnitt langgestreckten kreisförmigen Holzbriketts sowohl das handbetätigte Schlagwerkzeug 1 als auch die Spalterschütte 9.

In der Spalterschütte 9 kann ein noch unzerteiltes Holzbrikett 16 ortsfest eingelegt werden, mittels des Handwerkzeuges 1 gespaltet werden, wobei die hierdurch entstehende Holzspäne im Wesentlichen in der Spalterschütte 9 verbleiben. Nach der Entnahme der Holzbrikett-Stücke können die verbleibenden Spanreste durch die schräg angeordnete Vorderwandung 14 direkt aus der Spalterschütte 9 in einen Ofen geschüttet werden. Dieses System macht es somit möglich, in einem Wohnraum die Holzbriketts einfach, sauber und in der gewünschten Größe zu unterteilen. Zudem ist das ganze System sehr kompakt ausgebildet.

## Patentansprüche

1. Handbetätigtes Schlagwerkzeug zum Spalten von Holzbriketts umfassend einen aus Stahl oder Gusseisen gefertigten, balligen Griff (2) und zumindest eine Handschutzscheibe (4), die an einem Ende des Griffs (2) angebracht ist und ebenfalls aus Stahl oder Gusseisen gefertigt ist, sowie eine an der Handschutzscheibe (4) angebrachte, ballig-konische, in einer Spitze (6) auslaufenden Klinge (5), wobei das Schlagwerkzeug ein Gewicht von zumindest 300 g aufweist.

2. Schlagwerkzeug nach Anspruch 1,
**dadurch gekennzeichnet,**
**dass** das Schlagwerkzeug ein Gewicht von zumindest 350 g oder von zumindest 400 g oder von zumindest 450 g oder von zumindest 500 g oder von zumindest 550 g aufweist.

3. Schlagwerkzeug nach Anspruch 1 oder 2,
**dadurch gekennzeichnet,**
**dass** das Schlagwerkzeug ein Gewicht von nicht mehr als 1000 g oder von nicht mehr als 900 g oder von nicht mehr als 800 g oder von nicht mehr als 700 g aufweist.

4. Schlagwerkzeug nach einem der Ansprüche 1 bis 3, **dadurch gekennzeichnet, dass** der Griff (2) an seinen jeweiligen Enden mit Handschutzscheiben (3, 4) versehen ist.

5. Schlagwerkzeug nach einem der Ansprüche 1 bis 4 **dadurch gekennzeichnet, dass** die kurze Spaltklinge (5) pfeilförmig in der Spitze (6) zusammenläuft und zu seiner Basis hin ballig - konisch zunimmt, sowie die seitlichen Klingenkanten (7), ebenfalls von der Spitze zur Basis hin ballig - konisch verlaufend, hierbei zu zwei Schneidekanten (8) beidseitig geschliffen sind, wodurch sich eine scharfe, pfeilartige Spitze (6) ergibt, wobei die Spaltklinge (5) an ihrer ballig-konischen Basis um das ca. 0,2-fache seiner Länge zunimmt und zur Optimierung des Spaltvorganges eine Breite des ca. 0,7-fachen seiner ' Länge aufweist.

6. Schlagwerkzeug nach einem der Ansprüche 1 bis 5,
**dadurch gekennzeichnet,**
**dass** die Länge der Klinge (5) nicht mehr als 60 mm, insbesondere nicht mehr als 50 mm bzw. 45 mm beträgt und vorzugsweise nicht kürzer als 20 mm und insbesondere nicht kürzer als 30 mm bzw. 40 mm ist.

7. Schlagwerkzeug nach einem der Ansprüche 1 bis 6,
**dadurch gekennzeichnet,**
**dass** an einer Basis der Klinge (5), die mit der Handschutzscheibe (4) verbunden ist, die Klinge eine Bereite von 25 mm bis 45 mm und insbesondere von 30 mm bis 40 mm aufweist.

8. Schlagwerkzeug nach einem der Ansprüche 1 bis 7,
**dadurch gekennzeichnet,**
**dass** an einer Basis der Klinge (5), die mit der Handschutzscheibe (4) verbunden ist, die Klinge eine Dicke von 4 bis 10 mm und insbesondere 5 bis 8 mm aufweist.

9. Schlagwerkzeug nach einem der Ansprüche 1 bis 8,
**dadurch gekennzeichnet,**
**dass** die Handschutzscheibe (3, 4) einen Durchmesser von zumindest 50 mm aufweist.

10. Schlagwerkzeug nach einem der Ansprüche 1 bis 9,
**dadurch gekennzeichnet,**
**dass** die Handschutzscheibe (3, 4) eine Dicke von etwa zumindest 15 mm aufweist.

11. System zum Spalten von Holzbriketts umfassend
ein handbetätigtes Schlagwerkzeug nach einem der Ansprüche 1 bis 10 und eine Spalterschütte (9) zur Aufnahme eines Holzbriketts, wobei die Spalterschütte einen langgestreckten, wannenförmigen, einseitig offenen Hohlraum aufweist.

12. System nach Anspruch 11,
**dadurch gekennzeichnet, dass**
die Spalterschütte (9) zwei einander gegenüberliegend ausgebildete, schräg angeordnete Begrenzungswandungen (17) derart aufweist, dass ein im Querschnitt kreisförmiges Holzbrikett ortsfest in die Spalterschütte (9) einlegbar ist.

13. System nach Anspruch 11 oder 12,
**dadurch gekennzeichnet, dass**
die Spalterschütte (9) eine gegenüber einer Bodenwandung (10) schräg verlaufende Vorderwandung (14) aufweist, wobei zur Vorderwandung (14) gegenüberliegend eine Rückwandung (13) angeordnet ist, an welcher ein Griff (15) befestigt ist.

## Claims

1. Hand-operated striking tool for the chopping of wood briquettes, comprising a spherical handle (2) made of steel or cast iron, and at least one hand guard (4) which is attached to one end of the handle (2) and is also made of steel or cast iron, together with a spherical-conical blade (5) attached to the hand guard (4) and running to a point (6), wherein the striking tool has a weight of at least 300 g.

2. Striking tool according to claim 1,
**characterised in that**
the striking tool has a weight of at least 350 g or at least 400 g or at least 450 g or at least 500 g or at least 550 g.

3. Striking tool according to claim 1 or 2,
**characterised in that**
the striking tool has a weight of no more than 1000 g or no more than 900 g or no more than 800 g or no more than 700 g.

4. Striking tool according to any of claims 1 to 3
**characterised in that**
the handle (2) is provided with hand guards (3, 4) at each of its ends.

5. Striking tool according to any of claims 1 to 4, **characterised in that** the short chopping blade (5) comes to an arrow-shaped point (6) and increases conically towards its base, and the side blade edges (7), similarly spherical-conical from the point to the base, are sharpened to form two cutting edges (8) on either side, resulting in a sharp arrow- like point (6), wherein the chopping blade (5) increases at its spherical-conical base by around 0.2 times its length and to optimise the chopping process has a width of around 0.7 times its length.

6. Striking tool according to any of claims 1 to 5,
**characterised in that**
the length of the blade (5) is no more than 60 mm, in particular no more than 50 or 45 mm and preferably no shorter than 20 mm and in particular no shorter than 30 or 40 mm.

7. Striking tool according to any of claims 1 to 6,
**characterised in that**
at a base of the blade (5) which is joined to the hand guard (4), the blade has a width of 25 mm to 45 mm and in particular of 30 mm to 40 mm.

8. Striking tool according to any of claims 1 to 7,
**characterised in that**
at a base of the blade (5) which is joined to the hand guard (4), the blade has a thickness of 4 to 10 mm and in particular 5 to 8 mm.

9. Striking tool according to any of claims 1 to 8,
**characterised in that**
the hand guard (3, 4) has a diameter of at least 50 mm.

10. Striking tool according to any of claims 1 to 9,
**characterised in that**
the hand guard (3, 4) has a thickness of at least approximately 15 mm.

11. System for the chopping of wood briquettes comprising
a hand-operated striking tool according to any of claims 1 to 10 and a chopping container (9) for holding a wood briquette, wherein the chopping container has an elongated, trough-shaped hollow space, open at one side.

12. System according to claim 11,
**characterised in that**
the chopping container (9) has two opposite boundary panels (17), angled in such a way that a wood briquette of circular cross-section may be inserted immovably in the chopping container (9).

13. System according to claim 11 or 12,
**characterised in that** the chopping container (9) has a front panel (14) running at an angle to a base panel (10), wherein a rear panel to which a handle (15) is attached is positioned opposite the front panel (14).

## Revendications

1. Outil de percussion manuel pour fendre des briquettes de bois, comprenant un manche bombé (2) fabriqué en acier ou en fonte de fer, et au moins une plaque de protection pour la main (4), qui est rapportée à une extrémité du manche (2) et qui est également fabriquée en acier ou en fonte de fer, ainsi qu'une lame (5) rapportée sur la plaque de protection (4), de forme bombée-conique et se terminant dans une pointe (6), ledit outil de percussion ayant un poids d'au moins 300 g.

2. Outil de percussion selon la revendication 1,
**caractérisé en ce que** l'outil de percussion a un poids d'au moins 350 g ou d'au moins 400 g ou d'au moins 450 g ou d'au moins 500 g ou d'au moins 550 g.

3. Outil de percussion selon la revendication 1 ou 2,
**caractérisé en ce que** l'outil de percussion a un poids qui ne dépasse pas 1000 g ou ne dépasse pas 900 g ou ne dépasse pas 800 g ou ne dépasse pas 700 g.

4. Outil de percussion selon l'une des revendications 1 à 3, **caractérisé en ce que** le manche (2) est doté de plaques de protection pour la main (3, 4) à ses extrémités respectives.

5. Outil de percussion selon l'une des revendications 1 à 4, **caractérisé en ce que** la courte lame de fente (5) converge dans la pointe (6) à la manière d'une flèche, et augmente de manière bombée-conique en direction de sa base, de même que les arêtes latérales de la lame (7), qui s'étendent également sous forme bombée-conique depuis la pointe vers la base, sont ici meulées des deux côtés pour former deux arêtes de coupe (8), en raison de quoi il en résulte une pointe vive (6) semblable à une flèche, et la lame de fente (5) augmente au niveau de sa base bombée-conique à raison de 0,2 fois environ sa longueur et présente, pour optimiser l'opération de fente, une largeur d'environ 0,7 fois sa longueur.

6. Outil de percussion selon l'une des revendications 1 à 5,
**caractérisé en ce que** la longueur de la lame (5) ne dépasse pas 60 mm, en particulier ne dépasse pas 50 mm ou respectivement 45 mm, et n'est de préférence pas plus courte que 20 mm et en particulier pas plus courte que 30 mm ou respectivement 40 mm.

7. Outil de percussion selon l'une des revendications 1 à 6,
**caractérisé en ce que**, à une base de la lame (5), qui est reliée avec la plaque de protection pour la main (4), la lame présente une largeur de 25 mm à 45 mm, et en particulier de 30 mm à 40 mm.

8. Outil de percussion selon l'une des revendications 1 à 7,
**caractérisé en ce que**, à une base de la lame (5), qui est reliée avec la plaque de protection pour la main (4), la lame présente une épaisseur de 4 à 10 mm et en particulier de 5 à 8 mm.

9. Outil de percussion selon l'une des revendications 1 à 8,
**caractérisé en ce que** la plaque de protection pour la main (4, 3) présente un diamètre d'au moins 50 mm.

10. Outil de percussion selon l'une des revendications 1 à 9,
**caractérisé en ce que** la plaque de protection pour la main (3, 4) présente une épaisseur d'environ au moins 15 mm.

11. Système pour fendre des briquettes de bois, comprenant un outil de percussion manuel selon l'une des revendications 1 à 10, et une augette (9) pour recevoir une briquette de bois, dans lequel l'augette présente une cavité étirée en longueur, en forme de cuve et ouverte d'un côté.

12. Système selon la revendication 11,
**caractérisé en ce que** l'augette (9) comprend deux parois de délimitation (17) réalisées à l'opposé l'une de l'autre et agencées en oblique, de telle façon qu'une briquette de bois à section de forme circulaire peut être déposée de manière fixe dans l'augette (9).

13. Système selon la revendication 11 ou 12,
**caractérisé en ce que** l'augette (9) comprend une paroi antérieure (14) qui s'étend en oblique par rapport à une paroi de fond (10), et à l'opposé de la paroi antérieure (14) est agencée une paroi postérieure (13) à laquelle est fixée une poignée (15).
